# EUROPÄISCHE PATENTSCHRIFT

(11) **EP 0 773 827 B1**
(45) Veröffentlichungstag und Bekanntmachung des Hinweises auf die Patenterteilung: **13.10.1999**
(21) Anmeldenummer: 95926367.4
(22) Anmeldetag: 25.07.1995
(51) Int. Cl.: B01J 13/04, A61K 9/16

(54) **HERSTELLUNG VON ZELLEN ODER DISPERGIERTEN WIRKSTOFFEN IN MIKROVERKAPSELTER FORM SOWIE DERART HERGESTELLTE MIKROKAPSEL**
PREPARATION OF CELLS OR DISPERSED ACTIVE SUBSTANCES INTO MICRO-ENCAPSULATED FORM AND THUS OBTAINED MICRO-CAPSULES
PREPARATION DE CELLULES OU DE PRINCIPES ACTIFS DISPERSES SOUS FORME DE MICROCAPSULES, ET MICROCAPSULES AINSI OBTENUES

(30) Priorität: 26.07.1994 DE 4426396
(43) Veröffentlichungstag der Anmeldung: 21.05.1997
(73) Patentinhaber: Zimmermann, Ulrich, 97295 Waldbrunn (DE)
(72) Erfinder: Zimmermann, Ulrich, 97295 Waldbrunn (DE)
(74) Vertreter: Pöhner, Wilfried Anton, Dr.
(86) Internationale Anmeldenummer: DE9500972
(87) Internationale Veröffentlichungsnummer: WO9603205

(56) Entgegenhaltungen:
- EP-A- 0 167 690
- WO-A-85/05288
- WO-A-93/24112

## Beschreibung

Die Erfindung bezieht sich auf die Herstellung von Zellen oder dispergierten Wirkstoffen in mikroverkapselter Form hoher Konzentration mit einem Durchmesser von maximal 300 µm, bei der die Hülle den Kern unmittelbar anliegend umgibt und aus elastischem Material besteht, wobei nach verschiedenen Verfahren eine aus Hülle und dem die Zellen oder dispergierten Wirkstoffe aufnehmenden Kern aufgebaute Mikrokapsel hergestellt wird.

In der Medizin besteht das Bedürfnis, lebende Zellen in Form von Inseln in den lebenden menschlichen Körper einzubringen, die dort mit Nährstoffen versorgt werden und durch krankheitsbedingte Störungen fehlende Hormone produzieren und abgeben. Als Beispiel wird die Zuckerkrankheit (Diabetes mellitus) genannt, bei der insulinproduzierende Zellen in Form der sogenannten Langerhans'schen Inseln gewonnen und in die Leber oder den Pankreas appliziert werden. Zur Unterdrückung der Immunreaktionen ist es erforderlich, diese Zellen mit einer Hülle in Form einer Kapsel zu versehen, deren Kern sie dann bilden. Ein ähnliches Bedürfnis zur Verkapselung besteht bei unlöslichen jedoch dispergierten Wirkstoffen.

Verfahren zur Mikroverkapselung sind bekannt. Hierbei ist festzustellen, daß bei Kapseln hinreichend großer Durchmesser, d.h. von mehr als 600µm die Aufnahme eines Kernes ohne Probleme möglich ist. Bei demgegenüber wesentlich geringeren Durchmessern hingegen sind die meisten erzeugten Kapseln ohne Kern, d.h. leer. Eine weitere Anforderung ergibt sich daraus, daß die Anzahl der als Kern in die Kapsel einzubringenden Zellen oder Gewebestücke eine gewisse Größe nicht überschreiten dürfen, da dann deren innere Bereiche wegen Mangelversorgung absterben würden. Um den Bedarf des Körpers an den durch die Zellen erzeugten Hormone in ausreichendem Maße zu decken, ist Volumen oder Menge des einzubringenden Kernmateriales vorgegeben. Im Falle der Diabeteserkrankung müßte zur Erzeugung der notwendigen Insulinmenge etwa eine Million Langerhans'sche Inseln implantiert werden. Würde man hierzu Kerne großen Durchmessers einsetzen, müßte ein erhebliches die körperliche Verträglichkeit weit übersteigendes Volumen appliziert werden. Zu dem wäre die ansich gewünschte exakte Plazierung der Zellen aufgrund des hohen Eigenraumbedarfes fast nicht möglich. Ein weiterer Nachteil besteht darin, daß aufgrund der großen Diffusionswege zwischen der Hülle und dem Kern zusätzlich Versorgungsprobleme auftreten können. Aus den vorgenannten Gründen ist der Einsatz von Kapseln großen Durchmessers, d.h. von mehr als 600 µm in der Praxis ausgeschlossen. Die Verwendung von Kapseln mit kleinem Durchmesser bietet insofern ebenso keine Abhilfe, da die Reduzierung des Eigenvolumens einer einzigen Kapsel zur Folge hat, daß der Anteil der leeren, also ohne Kern versehenen Kapsel wesentlich zunimmt. Um die gleiche Menge an Zellen oder Wirkstoff einzubringen, tritt keine spürbare Verringerung des gesamten zu applizierenden Volumens ein. In diesem Fall würde ein großer Anteil an leeren und damit medizinisch inaktiven, andererseits jedoch nicht abbaubaren Material appliziert werden. Für die Anwendung von einer Million Langerhans'scher Inseln ist ein Eigenvolumen von etwa 100 bis 200 ml erforderlich.
Aus der EP-A-0 167 690 ist die Herstellung von mikroverkapselten Zellen oder Wirkstoffen bekannt, wobei allerdings zwischen Kern und der Hülle ausdrücklich ein Zwischenraum vorgesehen wird, um das aufgrund unterschiedlicher Einflüsse erfolgende Atmen des Kernes durch Änderungen des Durchmessers zuzulassen. Bei einem unmittelbaren Anliegen der Hülle würde aufgrund der dort verwendeten Materialien aufgrund ihrer unelastischen Eigenschaft bei Veränderung des Durchmessers des Kernes einer Zerstörung der Hülle erfolgen.

Hiervon ausgehend hat sich die Erfindung die Herstellung von Zellen oder dispergierenten Werkstoffen in mikroverkapselter Form zur Aufgabe gemacht, die bei hohem Zell- und Wirkstoffanteil ein Minimum an Eigenvolumen benötigen.

Gelöst wird die Aufgabe erfindungsgemäß dadurch, daß Luftdruck oder der Durchmesser der Kanäle in der Sprühdüse oder die Durchflußgeschwindigkeit der Lösung so eingestellt ist, daß das Volumen der Tropfen 1,5 - 2fache des Kernvolumens beträgt und anschließend einer Dichtezentrifuge zur Trennung zugeführt wird, deren Dichtegradient so eingestellt ist, daß die Dichte des eingesetzten Mediums höher als das der Hülle, jedoch niedriger als die Dichte der Hülle mit Kern gewählt ist. Die Hülle besteht aus Makromolekülen, die durch Vernetzung von aus den Monomeren Mannuron- und Guluronsäure bestehenden Polymeren mit Hilfe von Kationen gebildet sind (Alginat-Kationen-Komplex) und umschreibt ein Volumen, das den Kern unmittelbar umgibt und ist von einschichtigem Aufbau.

Der Kerngedanke der Erfindung besteht darin, entgegen der oben geschilderten und bekannten Probleme Kapseln mit kleinem Durchmesser von maximal 300 µm herzustellen und anschließend in einem Trennverfahren die leeren und somit biologisch inaktiven Kapseln zu trennen. Es findet eine Konzentration auf die mit Kernen bestückten kleinen Kapseln statt.

Die Herstellung der Kapseln kleinen Durchmessers geschieht in der Weise, daß eine mit zwei Kanälen versehene Sprühdüse eingesetzt wird, deren eine und zwar in Regel der außenliegende Kanal mit Luft beaufschlagt wird, der innere mit einer Lösung, die sowohl die Zelle oder die Wirkstoffe als auch den Hüllstoff also das Material für die Kapsel in löslicher Form enthält. Der Luftstrom bewirkt, daß die Tropfen bei einer gewissen Größe abgerissen werden. Die sich ablösenden Tropfen gelangen in ein Fällbad, wo sich die Hülle ausbildet. Durch entsprechende Variation des Luftdruckes, Wahl des Durchmessers des inneren Kanales der Sprühdüse als auch die Durchflußgeschwindigkeit der Lösung läßt sich erreichen, daß das Volumen der Tropfen etwa das 1,5 - 4fache des Kernvolumens bildet. Das Kernvolumen ist durch das Ausgangsmaterial bzw. das zu dessen Gewinnung angewandte Verfahren vorgegeben. Die Hülle umgibt den Kern unmittelbar und allseitig, ohne daß Hohlvolumina zurückbleiben, so daß die Tropfengröße das Volumen der Kapsel und aufgrund des vorgegebenen konstanten Kernvolumens auch das Volumen der Hülle bestimmt. Der Durchmesser der Kapsel beträgt maximal 300µm. Größere Durchmesser haben lange Diffusionswege zur Folge, die zu einer Mangelversorgung führen. Anschließend werden die ein Vielzahl an leeren Kapseln aufweisenden Reaktionsprodukte einer Dichtezentrifuge zugeführt, in der der Dichtegradient so eingestellt ist, daß die Dichte des Mediums höher ist als die des Hüllstoffes, jedoch niedriger als die Dichte der Hülle mit Kern. Somit erfolgt eine Separation der leeren Kapseln von den mit einem Kern bestückten.

Als konkretes Beispiel wird nachfolgend die Mikroverkapselung Langerhans'scher Inseln in Barium-Alginat-Mikrokapseln erläutert:

Für die Verkapselung von Langerhans'schen Inseln wurden Inseln aus männlichen Lewis Ratten (Charles River Wiga GmbH, Sulzfeld, Deutschland) mit einem Körpergewicht von mehr als 350 g durch intraductale Aufblähung und Collagenase Verdauung aus dem Pankreas herausgelöst und zweimal mit einer Lösung gewaschen, die 0,9 % NaCl und 10 mM Morpholinoethansulfonsäure enthielt (Zentrifugation 5 min bei 16 g).

Natrium-Alginat (Manugel GHB, Lot. No. 548643-567853, Kelco International Ltd., London) wurde 2,2%ig in destilliertem Wasser gelöst. Der pH-Wert der Alginat-Lösung wurde mit 1 N NaOH auf 7,0 - 7,4 eingestellt. Die Alginat-Lösung wurde durch einen Spitzenaufsatzfilter (0,2 µm; Renner, Darmstadt, Deutschland) sterilfiltriert. Dann wurden 9 ml des sterilfiltrierten Alginats mit 1 ml einer 9%igen NaCl-Lösung gemischt. In einem Milliliter dieser Lösung wurden ca. 1000 der (wie oben beschrieben) isolierten Langerhans'schen Inseln suspendiert. Zum Zertropfen der inselhaltigen Alginat-Lösung diente eine Dekordüse der Firma Sata Ludwigsburg, Deutschland). Diese besteht aus einem englumigen Hohlrohr (Düse, Lumendurchmesser: 0,35 mm), durch die die Flüssigkeit gedrückt wird, und einem Düsenkopf, der den Luftstrom um die Düse fokussiert. Düse und Düsenkopf waren an einem Stativ befestigt, an dem sich der Eingang für die Druckluft und die Alginat-Lösung befand. Mittels eines regelbaren, motorgetriebenen Kolbenvorschubs wurde die Alginat-Lösung aus einer 1-ml-Injektionsspritze durch den inneren Kanal der Sprühdüse gedrückt (0,2 ml/min). Mit Hilfe eines Druckluftstroms wurden von der Düse kleine Alginattröpfchen abgerissen. Die Druckluft wurde aus der zentralen Druckluftversorgung des Instituts entnommen, über einen Sterilfilter mit 0,2 µm Porenweite sterilisiert und mit Hilfe eines Vordruck- und eines Feindruckreglers auf einen Druck von 40 mbar eingestellt, wobei der Vordruck 1 bar betrug.

Aus der Düse tropfte die Alginat-Lösung in eine Petrischale (Durchmesser 10 cm), die 40 ml einer Lösung aus 20 mM Bariumchlorid, 0,72 % NaCl und 10 mM Morpholinoethansulsonsäure (pH 7) enthielt (= Fällbad). Der Abstand der Düse zum Fällbad betrug 10 cm. Beim Eintropfen in das Fällbad gelierte die Alginat-Lösung sofort und bildete Mikrokapseln mit einem Durchmesser von ca. 10 - 300 µm. Die meisten der entstandenen Mikrokapseln enthielten dabei keinen Kern (d.h. keine Inseln). Nach einer Verweilzeit von 10 - 15 min im Fällbad wurden die Mikrokapseln abzentrifugiert (3 min bei 170 g), dreimal mit 0,9%iger NaCl-Lösung gewaschen (Zentrifugation 3 min bei 170 g) und in 1 ml einer 0,9%igen NaCl-Lösung suspendiert.

Die leeren Mikrokapseln wurden durch diskontinuierliche Dichtegradientenzentrifugation von den inselhaltigen Mikrokapseln abgetrennt. Hierzu wurden unterschiedlich dichte Lösungen in einem Zentrifugenröhrchen übereinandergeschichtet. Diese Lösungen wurden durch Mischen einer Ficoll-Lösung (Biochrom, Berlin, Deutschland, Dichte 1,077 g/l bei 20° C) mit RPMI 1640 Flüssigmedium (Biochrom, Berlin, Deutschland) hergestellt. Die Lösungen wurden beginnend mit höchster Ficollkonzentration von unten nach oben in ein konisches 15-ml Zentrifugenröhrchen (Greiner, Nürtingen, Deutschland) pipettiert: Schicht 1: 1 ml Ficoll-Lösung, Schicht 2: 4 ml einer Lösung, die 6 Volumenteile Ficoll-Lösung und 4 Volumenteile RPMI 1640 enthielt, Schicht 3: 1 ml der Suspension der Mikrokapseln in 0,9%iger NaCl. Danach wurden die Röhrchen für 20 min bei 2700 g zentrifugiert. Die Zentrifugation erfolgte bei Zimmertemperatur mit ausgeschalteter Bremse. Nach der Zentrifugation befanden sich die leeren Kapseln auf Schicht 2, während die inselhaltigen Kapseln durch die Schicht 2 hindurchgewandert waren und sich auf Schicht 1 sammelten. Inseln, die nicht in eine Alginatkapsel eingeschlossen waren, konnten auch die Schicht 1 durchwandern und sedimentierten am Boden des Röhrchens. Durch ein Loch am Boden des Röhrchens wurde dann die Lösung abgelassen und in Portionen zu jeweils 0,5 ml aufgefangen. Die Portionen, die inselhaltige Mikrokapseln enthielten, wurden mit 20 - 30 ml 0,9iger NaCl verdünnt und die inselhaltigen Mikrokapseln für weitere Experimente durch Zentrifugation (20 min, 100 g) abgetrennt. Dieses Verfahren führte zu inselhaltigen Barium-Alginat-Mikrokapseln, die ein mittleres Volumen von 5,7 ± 2,2 x 10⁶ µm³ aufwiesen. Demgegenüber betrug das mittlere Volumen der unverkapselten Inseln 3,3 ± 1,4 x 10⁶ µm³.

Mehr als 90 % der zuletzt enthaltenen Mikrokapseln enthielten eine Insel. Von den ursprünglich eingesetzten Inseln wurden 72 % in mikroverkapselter Form zurückgewonnen.

Im Ergebnis erhält man eine Vielzahl an Kapseln kleinen Durchmessers, die aufgrund des Separationsverfahrens durchweg mit eine medizinisch aktiven Kern ausgerüstet sind. Deren Durchmesser ist geringfügig größer als der biologisch aktive Kern der Kapsel, so daß sich im Ergebnis das zu applizierende Volumen minimiert, d.h. nur geringfügig größer ist als das notwendigerweise einzubringende biologisch aktive und verwertbare Kernmaterial. Somit läßt sich die erforderliche Menge an biologisch aktivem Material auf ein Volumen von 5 bis 10 ml einschränken, das zielgerichtet und auch aufgrund des geringen Eigenvolumens genau plaziert werden kann. Aber auch in anderen Bereichen so z.B. für den Einsatz in Bioreaktoren eignen sich die erfindungsgemäß gewonnenen Substanzen zur Erzeugung hoher Leistungsdichte in erheblichem Umfang.

Neben dem beschriebenen und unter Anwendung von Luft und lösungsbeaufschlagten Düsen sind andere Methoden der Erzeugung der mikroverkapselten Zellen ode Wirkstoffe denkbar. Bei der elektrostatischen Herstellung von Mikrokapseln (electrostatic droplet generator) wird eine Suspension des zu verkapselnden Materiales, also des Kernmateriales, in einer wässrigen Lösung des Hüllmaterials hergestellt und durch eine Düse in ein Fällbad ausgetropft. Letztes enthält mehrwertige Kationen, durch die das Gelieren des Hüllmaterials ausgelöst wird. Entscheidend ist, daß zwischen der Düse und dem Fällbad ein in Fallrichtung wirkendes elektrostatisches Feld angelegt ist. Hierdurch wird bewirkt, daß das an der Düse austretende Tröpfchen eine hohe statische Aufladung erhält und aufgrund des entgegengesetzt geladenen Fällbades Anziehungskräften ausgesetzt ist, die zu einem frühzeitigen Abreißen des Tröpfchens führen. Das Ergebnis ist, daß vergleichsweise kleine Tropfen erzeugt werden. Im Gegensatz zum erstbeschriebenen Verfahren, bei dem das Abreißen der Tropfen mit Hilfe einer Luftströmung vorgenommen wird, bewirken hier elektrostatische Kräfte das frühzeitige Abreißen.

Das Beispiel einer Mikroverkapselung Langerhans'scher Inseln in Barium-Alginat-Mikrokapseln unter Verwendung einer elektrostatischen Tropfenbildung wird anschließend dargelegt:

Für die Verkapselung von Langerhans'schen Inseln wurden Inseln aus männlichen Lewis Ratten (Charles River Wiga GmbH, Sulzfeld, Deutschland) mit einem Körpergewicht von mehr als 350 g durch intraductale Aufblähung und Collagenase Verdauung aus dem Pankreas herausgelöst und zweimal mit einer Lösung gewaschen, die 0,9 % NaCl und 10 mM Morpholinoethansulfonsäure enthielt (Zentrifugation 5 mm bei 16g).

Natrium-Alginat (Manugel HB, Lot. No. 548643-567853, Kelco International Ltd., London) wurde 2,2%ig in destilliertem Wasser gelöst. Der pH-Wert der Alginat-Lösung wurde mit 1 N NaOH auf 7,0 - 7,4 eingestellt. Die Alginat-Lösung wurde durch einen Spritzenaufsatzfilter (0,2 µm; Renner, Darmstadt, Deutschland) sterilfiltriert. Dann wurden 9 ml des sterilfiltrierten Alginats mit 1 ml einer 9%igen NaCl-Lösung gemischt. In einem Milliliter dieser Lösung wurden ca. 1000 de (wie oben beschrieben) isolierten Langerhans'schen Inseln suspendiert. Die Alginat-Lösung wurde in eine 1 ml Injektionsspritze gefüllt, an die eine 22 G-Injektionsnadel angeschlossen war. Zum Zertropfen der inselhaltigen Alginat-Lösung diente ein elektrostatischer Tropfengenerator folgenden Aufbaus: Die Injektionsnadel war über ein Anschlußkabel mit dem negativen Ausgang eines Hochspannungs-Pulsgenerators verbunden. Genau 1 cm unter der Injektionsnadel befand sich eine Petrischale (Durchmesser 10 cm), die 20 ml einer Lösung aus 20 mM Bariumchlorid, 0,72 % NaCl und 10 mM Morpholinoethansulfonsäure (pH 7) enthielt (= Fällbad). Die Lösung in der Petrischale war mit dem positiven Ausgang des Pulsgenerators verbunden. Über einen regelbaren, motorgetriebenen Kolbenvorschub wurde die Alginat-Lösung mit einer Geschwindigkeit von 0,2 ml/min aus der Spritze herausgedrückt. Dabei wurden über den Hochspannungsgenerator alle 50 msec Pulse einer Feldstärke von 12 kV und einer Länge von 2 msec appliziert. Dadurch wurden von der Nadelspitze feinste Alginattröpfchen abgerissen, die in das Fällbad fielen. Beim Eintropfen in das Fällbad gelierte die Alginat-Lösung sofort und bildete Mikrokapseln mit einem Durchmesser von ca. 1 - 300 µm. Die meisten der entstandenen Mikrokapseln enthielten dabei keinen Kern (d.h. keine Inseln). Nach einer Verweilzeit von 10 - 15 min im Fällbad wurden die Mikrokapseln abzentrifugiert (3 min bei 170 g), dreimal mit 0,9%iger NaCl-Lösung gewaschen (Zentrifugation 3 min, bei 170 g) und in 1 ml einer 0,9%igen NaCl-Lösung suspendiert.

Die leeren Mikrokapseln wurden durch diskontinuierliche Dichtegradientenzentrifugation von den inselhaltigen Mikrokapseln abgetrennt. Hierzu wurden unterschiedlich dichte Lösungen in einem Zentrifugenröhrchen übereinandergeschichtet. Diese Lösungen wurden durch Mischen einer Ficoll-Lösung (Biochrom, Berlin, Deutschland, Dichte 1,077 g/l bei 20° C) mit RPMI 1640 Flüssigmedium (Biochrom, Berlin, Deutschland) hergestellt. Die Lösungen wurden beginnend mit höchster Ficollkonzentration von nach oben in ein konisches 15 ml-Zentrifugenröhrchen (Greiner, Nürtingen, Deutschland) pipittiert: Schicht 1: 1 ml Ficoll-Lösung, Schicht 2: 4 ml einer Lösung, die 6 Volumenteile Ficoll-Lösung und 4 Volumenteile RPPMI 1640 enthielt, Schicht 3: 1 ml der Suspension der Mikrokapseln in 0,9%iger NaCl. Danach wurden die Röhrchen für 20 min bei 2700 g zentrifugiert. Die Zentrifugation erfolgte bei Zimmertemperatur mit ausgeschalteter Bremse. Nach der Zentrifugation befanden sich die leeren Kapseln auf Schicht 2, während die inselhaltigen Kapseln durch die Schicht 2 hindurchgewandert waren und sich auf Schicht 1 sammelten. Inseln, die nicht in eine Alginatkapsel eingeschlossen waren, konnten auch die Schicht 1 durchwandern und sedimentierten am Boden des Röhrchens. Durch ein Loch am Boden des Röhrchens wurde dann die Lösung abgelassen und in Portionen zu jeweils 0,5 ml aufgefangen. Die Portionen, die inselhaltige Mikrokapseln enthielten, wurden mit 20 - 30 ml 0,9% NaCl verdünnt und die inselhaltigen Mikrokapseln für weitere Experimente durch Zentrifugation (20 min, 100 g) abgetrennt. Dieses Verfahren führte zu inselhaltigen Barium-Alginat-Mikrokapseln, die ein mittleres Volumen von 5,7 ± 2,2 x 10⁶ µm³ aufwiesen. Demgegenüber betrug das mittlere Volumen der unverkapselten Inseln 3,3 ± 1,4 x 10⁶ µm³. Mehr als 90 % der nach dem letzten Schritt erhaltenen Mikrokapseln enthielten eine Insel. Von den ursprünglich eingesetzten Inseln wurden 72 % in mikroverkapselter Form zurückgewonnen.

Bei dem weiterhin möglichen Verfahren der Emulsifikation wird ebenfalls zunächst eine Suspension des zu verkapselnden Materials (Zellen, Wirkstoffe) in einer wässrigen Lösung des Hüllmaterials vorgenommen. Anschließend wird diese wässrige Suspension in ein Ölbad oder in ein anderes, nicht mit Wasser mischbares Lösungsbad eingebracht. Durch intensives Rühren entsteht eine Wasser-in-Öl-Emulsion, deren Tropfengröße abhängig ist von der Intensität des Rührvorganges. Der Tropfendurchmesser wird um so kleiner, je intensiver der Rührvorgang erfolgt. Zur Einleitung des Gelierens des Hüllmaterials werden anschließend die mehrwertigen Kationen in das Bad eingebracht.

In einer Weiterbildung wird als zweckmäßig angesehen, daß hinausgehend über die oben beschriebene Trennung der leeren Kapseln von den übrigen zusätzlich noch eine Trennung des reinen Kernmaterials von dem in die Kapsel eingebrachten und einzig zur Applizierung bestimmten Fraktion erfolgt. Hierzu wird eine zusätzliche in Wirkrichtung der Zentrifugalkräfte nachgeordnete Trennstufe vorgesehen, wobei sich die Dichte der einzelnen Bereiche wie folgt verhält. Von der Drehachse, dem Ort der Probe, ausgehend wird eine Dichte gewählt, die größer ist als die der leeren Kapsel, jedoch niedriger als die der Kapsel mit Kern bzw. dem Kern alleine. In der nächsten Stufe wird eine Dichte gewählt, die niedriger ist als die des Kernes jedoch höher als die der Kapsel mit darin befindlichen Kern. Bei einer derartigen Vorgehensweise werden nicht nur die leeren Kapseln sondern auch die bloßen Kerne abgetrennt, so daß das tatsächlich gewünschte und zu gewinnen beabsichtigte Material, nämlich Kapseln mit Zellkern, zur Verfügung steht. Es läßt sich aus dem in radialer Richtung gesehenen mittleren Bereich gewinnen.

Der Dichteverlauf über den Radius kann stetig und kontinuierlich erfolgen. Bevorzugt ist jedoch, daß der Übergang zwischen den Bereichen unterschiedlicher Dichten stufenförmig erfolgt. In diesem Fall konzentrieren sich die zu gewinnen beabsichtigten Kapseln im Bereich der Stufe in hoher Konzentration, so daß bei der anschließend noch vorzunehmenden Extraktion eine maximale Ausbeute erreicht werden kann.

Als Material für die Hülle werden Alginat-Gele oder thermotrope oder ionotrope Gele vorgeschlagen. Die als "Alginat" bezeichneten Substanzen werden durch kettenförmige Mischpolymere gebildet, deren Elemente Guluron- und Mannuronsäuren sind. Alginat-Gele demgegenüber sind die zu einer netzartigen Struktur verbundenen kettenförmigen Mischpolymere des Alginats. Gele werden als thermotrop bezeichnet, wenn die Gelbildung durch eine Temperaturänderung induziert wird; ionotrope Gele hingegen bilden sich aus bei einer Änderung der Ionenkonzentration, wobei die die Gelbildung induzierende Ionenkonzentration in Abhängigkeit von der Struktur des Geles unterschiedlich sein kann. Als Beispiel für thermotrope Gele werden genannt Agar, Agarose, Gelatine und dergleichen. Als ionotrope Gele werden beispielhaft aufgeführt: Gellan, Carrageean sowie Cellulosesulfat.

Als Kernmaterial werden in der Regel lebende insbesondere Hormone produzierende aber auch tote Zellen oder dispergierte Wirkstoffe Einsatz finden. Entsprechend üblicher Terminologie werden als tote Zellen jene bezeichnet, deren Zellwandungen permeabilisiert sind. Dadurch werden die in den Zellen befindlichen Enzyme in die Lage versetzt, mit ihrer Umgebung ungehindert in Wechselwirkung zu treten und die gewünschten Umsetzungen vorzunehmen. Bei lebenden Zellen, also bei geschlossenen Zellmembranen, würde im Gegensatz hierzu der Umsatz nur sehr langsam bzw. überhaupt nicht erfolgen können. Der Begriff "Wirkstoffe" ist im Sinne der Erfindung allgemein auszulegen und umfaßt alle erdenklichen Arten von biologischer Wirkung zeigenden chemischen Verbindungen. Hierzu zählen insbesondere alle pharmakologischen, das heißt auf den menschlichen Organismus einwirkenden Wirkstoffe, als auch jene, auf Pflanzen (Herbizide) und auch auf Tiere (veterinärmedizinisch wirksame oder pestizide) Erzeugnisse.

Zur Vernetzung und Ausbildung der Hülle im Fällbad werden zweiwertige Bariumionen Ba++ und andere zwei- oder dreiwertige Kationen eingesetzt. Dies gilt insbesondere auch dann, wenn als Hüllstoff Alginat eingesetzt wird.

Bei der Verwendung von Alginat als Hüllstoff ist aus dem Stand der Technik bekannt, in das Fällbad Polykationen einzugeben. Es entsteht dann ein Alginat-Poly-Kationen-Komplex mit einem zweischichtigen Hüllenaufbau, da das Netz aus Alginat mit einem aus Polykationen überlagert wird. Dabei verbinden die Polykationen sowohl die Mannuron- als auch die Guluronsäure. Das erheblich nachteilige Ergebnis ist eine enge Maschenweite und eine hohe Dichte. Der entscheidende Nachteil ist, daß das Immunsystem diese Struktur als Antigen erkennt und entsprechende Reaktionen auslöst.

Die vorliegende Erfindung unterscheidet sich hiervon grundlegend, da zunächst ein einschichtiger Aufbau der Hülle entsteht, die als Alginat-Kationen-Komplex in aufbaumäßiger Hinsicht zu bezeichnen ist. Hierbei verbinden die Kationen die Mannuron- und Guluronsäuren. Es entsteht eine Vernetzung, bei der Alginat hinreichend niedriger Dichte ausgebildet wird. Die Dichte der Hülle ist somit wesentlich niedriger als die des Kernes, was in der nachfolgenden Abtrennung sich als entscheidender Vorzug erweist. Die Hülle entsteht durch ein Verbinden von Makromolekülen, die Polymere aus den beiden Monumeren Mannuron- und Guluronsäure darstellen. Somit entsteht der einschichtige Aufbau.

Die Dichte des Kerns ist immer größer als die Dichte der Hülle, da die Nährstoffe und Wirkstoffe hindurchdiffundieren müssen. Aufgrund der später vorzunehmenden Trennung ist ein großer Dichteunterschied zwischen Hülle und Kern von Vorzug. Obwohl die Dichte des Kernes Schwankungen unterliegt, muß die Dichte der Hülle stets größer gewählt werden. Bei der Kationenbindung gemäß der Erfindung entsteht eine Hülle vergleichsweise geringer Dichte, was als Vorteil zu werten ist. Andererseits darf die Dichte der Hülle nicht so gering gewählt werden, daß die Makromoleküle der Antikörper hindurchdiffundieren können. Eine Durchlässigkeit soll lediglich für die niedermolekularen Nähr- und Wirkstoffe gegeben sein. Daraus folgt, daß eine kleine Maschenweite, die eine bestimmte Größe nicht überschreiten darf, gefordert ist. Die Maschenweite wird über das Alginat eingestellt (im Stande der Technik durch die Wahl eines entsprechenden Polykations). Somit muß die Dichte der Hülle kleiner sein als die des Kernes, andererseits jedoch so groß gewählt werden, daß eine hinreichend geringe Maschenweite entsteht, die ein Hindurchdiffundieren der Antikörper unterbindet.

Vorliegende Erfindung beansprucht eine Mikrokapsel, in deren Inneren Zellen oder dispergierte Wirkstoffe als Kern untergebracht sind. Deren Außendurchmesser beträgt maximal 200 bis 300 µm. Bei größeren Durchmessern werden die Diffusionswege zu lang, so daß die Kerne nicht mehr ausreichend mit Nährstoffen versorgt werden können. Der Kern ist allseitig und unmittelbar, d.h. ohne daß dazwischen Hohlräume befindlich sind, mit der Hülle umgeben. Das Kernvolumen ist vorgegeben durch das einzubringende Material und wird in seinen Abmessungen bestimmt durch die jeweils angewandte Gewinnungsmethode. Je größer die Tropfengröße eingestellt ist, umso größer wird das Hüllvolumen. Verfahren, mit deren Hilfe derartige Mikrokapseln herstellbar sind, wurden oben bereits dargestellt.

Bei Verwendung von Alginaten mit Kationen entsteht eine Hülle aus Makromolekülen, bei denen Polymere durch Kationen vernetzt sind. Diese Polymeren entstehen aus monumeren der Mannoron- und Gulloronsäure.

## Patentansprüche

1. Herstellung von Zellen oder dispergierten Wirkstoffen in mikroverkapselter Form hoher Konzentration mit einem Druchmesser von maximal 300 µm, bei der die Hülle den Kern unmittelbar anliegend umgibt und aus elastischem Material besteht, wobei mit Hilfe einer Spritzdüse aus zwei Kanälen, die einerseits durch Luft und andererseits durch ein Gemisch einer Lösung der Zellen oder Dispersion des Wirkstoffes mit dem Hüllstoff in löslicher Form beaufschlagt werden, so daß Tropfen entstehen, die in ein Fällbad gelangen, wo sich die Hülle ausbildet, **dadurch gekennzeichnet,** daß Luftdruck oder der Durchmesser der Kanäle in der Sprühdüse oder die Durchflußgeschwindigkeit der Lösung so eingestellt ist, daß das Volumen der Tropfen das 1,5 - 4fache des Kernvolumens beträgt und anschließend einer Dichtezentrifuge zur Trennung zugeführt wird, deren Dichtegradient so eingestellt ist, daß die Dichte des eingesetzten Mediums höher als das der Hülle, jedoch niedriger als die Dichte der Hülle mit Kern gewählt ist und daß die Hülle aus Makromolekülen besteht, die durch Vernetzung von aus den Monomeren Mannuron- und Guluronsäure bestehenden Polymeren mit Hilfe von Kationen gebildet sind (Alginat-Kationen-Komplex) und ein Volumen umschreibt, das den Kern unmittelbar umgibt und von einschichtigem Aufbau ist.

2. Herstellung von Zellen oder dispergierten Wirkstoffen in mikroverkapselter Form hoher Konzentration mit einem Druchmesser von maximal 300 µm, bei der die Hülle den Kern unmittelbar anliegend umgibt und aus elastischem Mateiral besteht, wobei aus einer Düse ein Gemisch einer Lösung der Zellen oder Dispersion des Wirkstoffes mit dem Hüllstoff tropfenweise in ein Fällbad abgegeben werden, wo sich die Hülle ausbildet und zwischen Fällbad und Düse ein elektrostatisches Feld anliegt, **dadurch gekennzeichnet,** daß die Feldstärke des elektrischen Feldes so eingestellt wird, daß das Volumen der Tropfen das 1,5 - 4fache des Kernvolumens beträgt und anschließend einer Dichtezentrifuge zur Trennung zugeführt wird, deren Dichtegradient so eingestellt ist, daß die Dichte des eingesetzten Mediums höher als das der Hülle, jedoch niedriger als die Dichte der Hülle mit Kern gewählt ist und daß die Hülle aus Makromolekülen besteht, die durch Vernetzung von aus den Monomeren Mannuron- und Guluronsäure bestehenden Polymeren mit Hilfe von Kationen gebildet sind (Alginat-Kationen-Komplex) und ein Volumen umschreibt, das den Kern unmittelbar umgibt und von einschichtigem Aufbau ist.

3. Herstellung von Zellen oder dispergierten Wirkstoffen in mikroverkapselter Form hoher Konzentration mit einem Druchmesser von maximal 300 µm, bei der die Hülle den Kern unmittelbar anliegend umgibt und aus elastischem Mateiral besteht, wobei eine Suspension der zu verkapselnden Zellen oder Wirkstoffe in einer wässrigen Lösung des Hüllmaterials vorgenommen wird und die Suspension in ein Ölbad oder ein anderes nicht mit Wasser mischbares Lösungsbad eingebracht wird und anschließend ein Rührvorgang durchgeführt wird, **dadurch gekennzeichnet,** daß die Intensität des Rührvorganges so eingestellt wird, daß das Volumen der Tropfen das 1,5 - 4fache des Kernvolumens beträgt, dann mehrwertige Kationen zum Ausfällen des Hüllmaterials in das Bad eingebracht werden und anschließend einer Dichtezentrifuge zur Trennung zugeführt wird, deren Dichtegradient so eingestellt ist, daß die Dichte des eingesetzten Mediums höher als das der Hülle, jedoch niedriger als die Dichte der Hülle mit Kern gewählt ist und daß die Hülle aus Makromolekülen besteht, die durch Vernetzung von aus den Monomeren Mannuron- und Guluronsäure bestehenden Polymeren mit Hilfe von Kationen gebildet sind (Alginat-Kationen-Komplex) und ein Volumen umschreibt, das den Kern unmittelbar umgibt und von einschichtigem Aufbau ist.

4. Verfahren nach einem der Ansprüche 1 bis 3, **dadurch gekennzeichnet,** daß in Richtung der Beschleunigungskräfte zwei Trennstufen vorgesehen sind, wobei die erste dadurch gebildet wird, daß sich an einen Bereich mit einer höheren Dichte als dem der Hülle eine mit einer niedrigen Dichte als Hülle mit Kern oder dem Kern anschließt und weiterhin in Wirkung der Zentrifugalkräfte einen Bereich mit höherer Dichte als Hülle mit Kern, jedoch niedrigere Dichte als Kern anschließt.

5. Verfahren nach Anspruch 4, **dadurch gekennzeichnet,** daß die Änderung der Dichte des eingesetzten Materiales über den Radius stufenförmig erfolgt.

6. Verfahren nach einem der Ansprüche 1 bis 5, **dadurch gekennzeichnet,** daß das Material der Hülle Alginat-Gele oder thermotrope oder ionotrope Gele sind.

7. Verfahren nach einem der Ansprüche 1 bis 6, **dadurch gekennzeichnet,** daß zur Ausfällung zwei- oder dreiwertige Kationen, insbesondere Ba++-Ionen eingesetzt werden.

8. Verfahren nach einem der Ansprüche 1 bis 7, **dadurch gekennzeichnet,** daß als Kern lebende, insbesondere hormonerzeugende oder tote Zellen oder dispergierte Wirkstoffe eingesetzt werden.

9. Mikroverkapselte Zellen oder dispergierte Wirkstoffe mit einem Kern aus toten oder lebenden Zellen oder dispergierten Wirkstoffen, dessen Durchmesser maximal 300 µm beträgt, **gekennzeichnet durch** eine Hülle, die aus Makromolekülen besteht, die durch Vernetzung von aus den Monomeren Mannuron- und Guluronsäure bestehenden Polymeren mit Hilfe von Kationen gebildet sind (Alginat-Kationen-Komplex) und ein Volumen umschreibt, das etwa das 1,5 - 4fache des Kernvolumens beträgt, den Kern unmittelbar umgibt und von einschichtigem Aufbau ist.

## Claims

1. Preparation of cells or dispersed active substances into high-concentration microcapsules with a maximum diameter of 300 µm, the shell of which surrounds directly the core and consists of an elastic material, where with the aid of a spray nozzle comprising two channels, which are impinged on the one hand by air and on the other hand by a mixture of a solution of the cells or dispersion of the active substance with the shell material in soluble form, droplets are created which reach a precipitation bath where the shell is formed, **wherein** air pressure or the diameter of the channels in the spray nozzle or the flow rate of the solution is set so that the droplet volume is 1.5 to 4 times the core volume and is then supplied to a density centrifugal separator with a density gradient adjusted so that the density of the used medium is higher than that of the empty microcapsule shells but lower than the density of the core-containing shells and the shell consists of macromolecules which are formed by cross-linking polymers consisting of the monomers mannuronic and guluronnic acid with the aid of cations (alginate-cation complex) and circumscribes a volume that surrounds the core directly and is a single-layer structure.

2. Preparation of cells or dispersed active substances into high-concentration microcapsules with a maximum diameter of 300 µm, the shell of which surrounds directly the core and consists of an elastic material, in which from one nozzle a mixture of a solution of the cells or dispersion of the active substance with the shell material is delivered droplet-wise into a precipitation bath, where the shell forms, and an electrostatic field is applied between the precipitation bath and the nozzle, **wherein** the field strength of the electric field is adjusted so that the volume of the droplets is 1.5 to 4 times the core volume and is then supplied to a density centrifugal separator with a density gradient adjusted so that the density of the used medium is higher than that of empty microcapsule shells but lower than the density of core-containing shells and the shell consists of macromolecules which are formed by cross-linking polymers consisting of the monomers mannuronic and guluronnic acid with the aid of cations (alginate-cation complex) and circumscribes a volume that surrounds the core directly and is a single-layer structure.

3. Preparation of cells or dispersed active substances into high-concentration microcapsules with a maximum diameter of 300 µm, the shell of which surrounds directly the core and consists of an elastic material, in which the cells to be encapsulated are prepared in a suspension or the active substances to be encapsulated in an aqueous solution of the shell material and the suspension is introduced into an oil bath or another solution bath which cannot be mixed with water and then agitated, **wherein** the intensity of the agitation process is adjusted so that the volume of the droplets is 1.5 to 4 times the core volume, then polyvalent cations are introduced to the bath to precipitate the shell material and the microcapsules are then supplied to a density centrifugal separator with a density gradient adjusted so that the density of the used medium is higher that of the empty microcapsule shells but lower than the density of the core-containing shells and the shell consists of macromolecules which are formed by cross-linking polymers consisting of the monomers mannuronic and guluronnic acid with the aid of cations (alginate-cation complex) and circumscribes a volume that surrounds the core directly and is a single-layer structure.

4. Process according to one of claims 1 to 3, **wherein** in the direction of the accelerating force two separation walls are provided, the first being formed in that a region with a higher density than that of the shell adjoins one with a lower density than the shell with core or the core and further in the action of the centrifugal force adjoins a region of higher density than the shell with core but lower density than the core.

5. Process according to claim 4, **wherein** the change of density of the used material is effected step-wise across the radius.

6. Process according to one of claims 1 to 5, **wherein** the material of the capsule comprises alginate gels or thermotropic or ionotropic gels.

7. Process according to one of claims 1 to 6, **wherein** bivalent or trivalent cations, especially Ba++ ions are used for precipitation.

8. Process according to one of claims 1 to 7, **wherein** living, especially hormone generating or dead cells or dispersed active substances are used as the core.

9. Micro-encapsulated cells or dispersed active substances having a core of dead or living cells or dispersed active substances, whose maximum diameter is 300 µm, **wherein** a shell, which consists of macromolecules, which are formed by cross-linking polymers consisting of the monomers mannuronic and guluronnic acid with the aid of cations (alginate-cation complex) and circumscribes a volume which is more or less 1.5 to 4 times the volume of the core, surrounds the core directly and is a single-layer structure.

## Revendications

1. Fabrication de cellules ou de substances actives sous forme de microcapsules de forte concentration, d'un diamètre maximal de 300 µm, dans laquelle la gaine protectrice, réalisée dans une matière élastique, colle directement au noyau, une buse composée de deux conduits dont l'un est alimenté en air comprimé et l'autre en mélange composé d'une solution des cellules ou d'une dispersion de substances actives et de la matière de la gaine sous forme soluble, formant des gouttes qui tombent dans un bain de précipitation dans lequel se forme la gaine, **caractérisée en ce que** le courant d'air comprimé ou la vitesse d'écoulement de la solution est réglé de sorte que le volume des gouttes représente 1,5 à 4 fois le volume du noyau, et en ce que l'on effectue ensuite une séparation à l'aide d'une centrifugeuse par densité, réglée de telle sorte que la densité du milieu utilisé est supérieure à celle de la gaine, mais inférieure à la densité de la gaine avec le noyau, et en ce que la gaine, formée de macromolécules résultant de la réticulation des polymères composés des acides monomères mannurone et gulurone, déclenchée à l'aide de cations (complexe alginate-cations), délimite un volume enveloppant directement le noyau, et se compose d'une seule couche.

2. Fabrication de cellules ou de substances actives sous forme de microcapsules de forte concentration, d'un diamétre maximal de 300 µm, dans laquelle la gaine protectrice, réalisée dans une matière élastique, colle directement au noyau, une buse injectant un mélange composé d'une solution renfermant les cellules ou la dispersion et de la matière de la gaine goutte à goutte dans un bain de précipitation dans lequel se forme la gaine, et un champ électrostatique étant appliqué entre le bain de précipitation et la buse, **caractérisée en ce que** l'intensité du champ électrostatique est réglée de telle sorte que le volume des gouttes représente 1,5 à 4 fois le volume du noyau et que l'on utilise ensuite une centrifugeuse pour effectuer une séparation par densité, le degré de densité étant réglé de sorte que la densité du milieu utilisé est supérieure à celle de la gaine, mais inférieure à la densité de la gaine avec le noyau, et en ce que la gaine, formée de macromolécules résultant de la réticulation des polymères composés des acides monomères mannurone et gulurone, déclenchée à l'aide de cations (complexe alginate-cations), délimite un volume entourant directement le noyau et se compose d'une seule couche.

3. Fabrication de cellules ou de substances actives sous forme de microcapsules de forte concentration, d'un diamètre maximal de 300 µm, dans laquelle la gaine protectrice, réalisée dans une matière élastique, colle directement au noyau, une suspension renfermant les cellules ou les substances actives à encapsuler dans une solution aqueuse de la matière de la gaine étant introduite dans un bain d'huile ou dans un autre bain non miscible à l'eau, puis agitée, **caractérisée en ce que** l'intensité du processus d'agitation est réglée de telle sorte que le volume des gouttes représente 1,5 à 4 fois le volume du noyau, et en ce que l'on introduit des cations polyvalents dans le bain pour déclencher la précipitation de la matière de la gaine, et que l'on utilise ensuite une centrifugeuse pour effectuer une séparation par densité, le degré de densité étant réglé de telle sorte que la densité du milieu utilisé est supérieure à celle de la gaine, mais inférieure à la densité de la gaine avec le noyau, et en ce que la gaine, formée de macromolécules résultant de la réticulation des polymères composés des acides monomères mannurone et gulurone, déclenchée à l'aide de cations (complexe alginate-cations), délimite un volume entourant directement le noyau et se compose d'une seule couche.

4. Procédé selon l'une des revendications 1 à 3, **caractérisé en ce que** on prévoit deux paliers de séparation dans le sens des forces d'accélération, le premier étant formé par la zone de densité supérieure à la zone de la gaine suivie d'une zone de densité inférieure à celle de la gaine avec le noyau ou du noyau seul, et étant suivi, toujours dans le sens d'action des forces centrifuges, par une zone de densité supérieure à celle de la gaine avec le noyau, mais inférieure à celle du noyau seul.

5. Procédé selon la revendication 4, **caractérisé en ce que** la variation de la densité de la matière utilisée évolue par paliers le long du rayon.

6. Procédé selon l'une des revendications 1 à 5, **caractérisé en ce que** la matière de la gaine est un gel d'alginate ou un gel thermotrope ou ionotrope.

7. Procédé selon l'une des revendications 1 à 6, **caractérisé en ce que** l'on utilise des cations bivalents ou trivalents, notamment des ions Ba++ pour déclencher la précipitation.

8. Procédé selon l'une des revendications 1 à 7, **caractérisé en ce que** l'on utilise des cellules vivantes, et notamment des cellules produisant des hormones, ou des cellules mortes, ou encore des substances actives dispersées comme noyau.

9. Cellules ou substances actives dispersées, microencapsulées, avec un noyau constitué de cellules mortes ou vivantes ou de substances actives dispersées, **caractérisées en ce que** une gaine, formée de macromolécules obtenues par réticulation des polymères composés des acides monomères mannurone et gulurone, déclenchée à l'aide de cations (complexe alginate-cations), délimite un volume représentant environ 1,5 à 4 fois le volume du noyau, colle directement à celui-ci, et se compose d'une seule couche.
